(19) Europäisches Patentamt
European Patent Office
Office européen des brevets

(11) **EP 4 681 718 A1**

(12) **EUROPEAN PATENT APPLICATION**

(43) Date of publication:
21.01.2026 Bulletin 2026/04

(21) Application number: 24020241.6

(22) Date of filing: **19.07.2024**

(51) International Patent Classification (IPC):
*A61K 33/00* (2006.01)   *A61K 33/14* (2006.01)
*C01D 15/00* (2006.01)   *C22B 26/00* (2006.01)
*B01D 61/00* (2006.01)   *B01F 25/00* (2022.01)
*C01D 3/00* (2006.01)

(52) Cooperative Patent Classification (CPC):
**A61K 33/00; A61K 33/14; B01F 25/00; C01D 3/00; C01D 15/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Ponzini, Sergio**
**90047 Partinico (PA) (IT)**

(72) Inventor: **Ponzini, Sergio**
**90047 Partinico (PA) (IT)**

(54) **LITHIUM ENRICHED WATER AND ITS PREPARATION FOR USE**

(57)    Lithium is an alkaline earth metal, long used in psychiatric pharmacology as a mood stabilizer in the treatment of bipolar disorder (BD). The therapeutic dosage of lithium carbonate is calibrated so as to have a lithiaemia in a patient with BD between 0.4 and 0.8 mmol/liter.

However, several studies have shown that at lower doses lithium has an anti-aging effect while maintaining the functionality or enhancing the functional capacity of target organs. There are mineral waters on the market that are naturally enriched with lithium, thanks to their path in the subsoil, but generally the final concentration of lithium is much lower than that capable of exhibiting detectable biological effects.

To date, there are no drinks artificially enriched with lithium and there is also no industrial process that artificially adds lithium to drinks and waters aimed at obtaining an effective dose following consumption.

This invention concerns a new product that includes a drink artificially enriched with lithium suitable for human consumption and an example of the process aimed at its preparation.

**EP 4 681 718 A1**

**Description**

The purpose of the invention is the production of a new product that includes a water artificially enriched with lithium suitable for human consumption and the description of a process aimed at its preparation, such as to guarantee psychophysical benefits, without side effects, drinkable by all.

In many studies it has been seen that, at various subtherapeutic doses, lithium does not produce side effects but benefits the organs. For example, on the kidney, the psychiatric dosage of lithium can be nephrotoxic while, at subtherapeutic dosage, it is nephroprotective. A therapeutic effect of lithium at those doses has even been demonstrated on acute renal lesions AKI and on glomerular lesions by decreasing proteinuria and acting on the cells of the glomerular filter, the podocytes. In particular, an experiment on mice shows that, at serum lithium concentrations of 0.25 mM, lithium was nephroprotective, that is, it defends the kidney from oxidative stress resulting from normal (aging) or pathological organ function. At a concentration of 1 mM, however, it caused dose-dependent renal failure (Riadh Nciri et oll 2008). Another study shows that lithium administered to mice at a dose of 40 mg/kg as a single dose enhances renal recovery and nephron repair through duplication of surviving epithelial cells and that the mechanism passes through the inhibition of GSK3B. (Hui Bao 2014). Indeed, a very recent study (Yudong 2022) demonstrates how the overexpression of GSK3B correlated with age causes renal aging (in terms of podocytes and therefore of glomerular filter) and that therefore lithium (GSK3B inhibitor) determines a nephroprotection that is obviously also of a functional type. This anti-aging effect on the kidney would increase longevity. A study demonstrates that the repairing effect on the kidney during Aki is mainly of a compensatory type of the surviving podocytes, because lithium, by inhibiting GSK3B, influences the microtubular and therefore cytoskeletal structure of the podocytes that increase in volume and lengthen their glomerular processes (WeiWei 2014) but also occurs by decreasing podocyte motility (WeiWei 2014). Therefore, studies on the kidney generally demonstrate that lithium, at subtherapeutic doses, by inhibiting GSK3B, increases qualitative maintenance and functional capacity over time and has a repairing effect in the course of renal injury. The dose-dependent effect has also been demonstrated in an article (2016 by Jaining Llu) which, starting from small doses of lithium and gradually increasing them, noted a proliferative effect on the cells of the metanephric mesenchyme at 10.20 30.40 mM and toxic starting from 50 mM (Jaining Liu2016).

On the bone, lithium at a dose of 20 mg/kg has been shown to strengthen the endochondral ossification process, to speed up the repairing process in the case of fracture with formation of bone callus and to protect against osteoporosis or delay its onset (Kathak Vachhani 2017). This effect on the bone derives from osteoblastic activation and osteoclast suppression (ColineHaxaire 2016). These results were confirmed in experiments on ovariectomized mice to experimentally induce osteoporosis. (Jianhai Bai 2019).

Surprising effects at subtherapeutic doses of lithium are also demonstrated on the cardiovascular system. In particular, on the vessels, at a concentration of 0.2 -0.4 mmol/l, they demonstrate good vascular relaxing capabilities, even if the lowering of pressure is not significant, and the ability to stabilize the endothelium and lower permeability even at rest, this last data is fundamental also in the course of cardiovascular and cerebrovascular diseases as in the course of both ischemic and hemorrhagic stroke. Lithium therefore reduces the risk of stroke and, if such an event is in progress, reduces brain damage due to its stabilizing effect on the endothelium and its effect of lowering permeability both at rest and in dynamic situations, thus demonstrating an anti-edema effect (Bert Bosche 2016). On the heart, experiments with serum lithium dosage of 0.39 mM demonstrate a positive physiological hypertrophic effect and, at the same time, this dosage inhibits pathological cardiac remodeling by moving away from the dilated phase of the heart and therefore demonstrating an anti-heart failure effect (TsengMing Lee 2017). Another experiment on mice fed low-dose lithium via drinking water with a concentration of 10 mg / kg confirms the data demonstrating that lithium at subtherapeutic dosages prevents dilated cardiopathy and heart failure (Sofial. Hamstra 2020).

In skeletal muscle, it has been shown that, in cell culture, at a concentration of 0.5 mM, lithium facilitates the fusion of myoblasts into muscle cells and therefore myogenic differentiation (NigelKurgan 2019).

Lithium in mice (at a dose of 10 mg / kg per day in mice, which resulted in a serum concentration of 0.02 mmol / l) increases skeletal muscle hypertrophy, improves resistance to fatigue and increases strength (Kennedy C. Whitley 2020).

Furthermore, with low-dose lithium supplementation, it has been shown that the formation of brown fat is promoted in the muscle, useful for muscle thermogenesis and at the same time useful for improving conditions of obesity and dysmetabolic conditions including diabetes (Mia S Geromella 2022).

A 2011 study had already highlighted the anti-diabetic role of lithium and had shown that insulin resistance decreased precisely thanks to the inhibition of GSK3B.

In the brain, data on lithium in bipolar disorder had shown that the metal increased gray matter especially in the hippocampal area, stimulating the proliferation of neuronal stem cells whose number also depends on age. These results of brain enhancement, but also neuroprotection, depend on the inhibition of GSK3B. In fact, it is seen in experiments on animals but also in culture that in the presence of lithium, free radicals and oxidative stress in general decrease, brain growth factors increase, proliferative pathways are stimulated and cell death pathways (apoptosis) are inhibited, cerebral excitotoxicity from glutamate decreases. Therefore, it is thought that subtherapeutic doses of lithium but at higher doses than the concentration of lithium in commercial bottles of natural water, can prevent neurodegenerative diseases such as Alzheimer's and Parkinson's, also because it has been seen that lithium allows the non-accumulation of amyloid plaques such as tau in the brain, and that in general it prevents the cognitive deterioration of dementia (Carol A Lazzara 2015). Lithium has demonstrated a neuroprotective effect at a serum dosage of 0.2 mmol/l and has also demonstrated an anti-aggressive effect in patients with Alzheimer's at serum dosages between 0.3 - 0.8 mM (DP Devanand 2018).

It has also been shown that lithium, in rats, protects, if taken chronically, from ischemic liver damage (ANding Liu 2013).

Lithium, if taken chronically and at low doses, has antineoplastic effects especially on prostate cancer (Benyi Li 2016), where the combined treatment of lithium and antineoplastics in metastases from androgen-dependent prostate cancer is already in the experimental phase (Vajihe Azimian Zavareh 2012). On pancreatic adenoma, lithium activates apoptotic signals in pancreatic tumor cells and decreases their tumorigenic potential (Zhonglu Peng 2013). Antineoplastic effects have been demonstrated on NET (neuroendocrine tumors) (Sam J Lubner 2011), on esophageal squamous cell carcinoma, where lithium suppresses its progression, on colon carcinoma, on small cell lung cancer, on osteoma and rhabdomyosarcoma, on leukemia. The inhibition of GSK3B carried out by lithium facilitates the maturation of NK cells and increases the cytotoxic effect of CD8 T lymphocytes clearly important in counteracting the onset of potential tumor cells (Takahiro Domoto 2020).

Many studies affirm a relationship between lithium levels, longevity and mental health in general. The theory is that low-dose lithium improves connectivity between neurons and exposure throughout life increases the perception of well-being (Laurence Kavanagh 2017).

Literature data on lithium consumption from mineral waters suggest that the best dosage is 1 mg / liter which can induce a serum concentration in those who drink it between 0.1 and 0.2 mmol / l even in exceptional cases such as certain diets and severe dehydration, lithium levels were to rise due to accumulation, they would always remain below 0.3 mmol / l, i.e. at subtherapeutic dosages and beneficial for tissues.

The invention exploits the fact that lithium can be artificially added to water, through industrial processes, all aimed at human consumption. The lithium source can be natural or artificial, but typically the addition of lithium occurs through the dissolution of lithium chloride salts until the desired concentration is reached, generally greater than 0.05 mg/liter, between 0.1 mg/liter and 10 mg/liter, preferably 1 mg/liter. The product is suitable for human consumption as set out above, for example as an anti-aging formulation and as protective water for target organs.

The invention consists in artificially enriching water with lithium, bringing it to an ideal dosage to allow the diffusion of its beneficial effects and therefore improve the quality of bottled water.

An example of an industrial process of lithium addition via lithium chloride involves the use of the parallel chamber system: two chambers of equal volume V separated by an inverted V tube, equipped with two valves at the beginning of the branching of the original conduit. The two tanks are equipped with a lithium chloride pump or injector whose dosage is in relation to the volume of the two tanks, knowing that for each litre of natural mineral water 8.47 mg of lithium chloride must be dissolved. The lithium salt (solubility of lithium chloride at 20 degrees centigrade is 832 g/l) will be added alternately to one of the two chambers, so that when one is already completely filled and begins to empty, the other begins to fill. All this is possible thanks to the opening / closing of the 4 valves respectively 2 upstream and 2 downstream of the two chambers. The position of the valves in the individual ducts, the first ones that carry water entering the two chambers and the second ones that carry the flow of water exiting the two ducts that converge in a single duct, ensures that the process is continuous and not limiting. The solvation phase of lithium chloride to give chloride ions and lithium ions is an exergonic process, therefore with a negative enthalpy difference dH. The solubilization reaction of lithium chloride is guaranteed by the rotating action of the helical axis present in the two chambers. The addition of lithium chloride via an injector will take place at the top of each of the two chambers and during the filling process of the chambers. To ensure that the process is continuous and functional, it is necessary to place a high-flow pump between each chamber and the exit duct. The two pumps will activate alternately during the emptying phases of each chamber, removing water and ensuring that the filling phase of one chamber has the same duration as the emptying phase of the other chamber and vice versa. By doing so, the valve upstream of each chamber will open at the same time as the valve downstream of the other chamber opens and the valve downstream of one chamber will close at the same time as the valve upstream of the other chamber closes and the industrial process will be able to proceed regularly.

The need for a high-capacity pump arises from the fact that the filling pressure of the chambers is greater than the emptying pressure. The latter, in fact, depends exclusively on the force of gravity, the height of the fluid and the density of the water since the fluid has been broken by the closing of the upstream valves. In particular, the characteristics of the hydraulic system can be derived from Bernoulli's equation and the industrial process can be made continuous:

$$1/2 \varrho v_1^2 + \varrho g h + P_1 = 1/2 \varrho v_2^2 + \varrho g h + P_2$$

continuity equation

$$Av_1 = Av_2$$

An alternative process (no longer a continuous process but an intermittent industrial process) can be obtained by lowering the incoming pressure, changing the timing of the closing/opening of the valves, and/or decreasing the pressure of the high-flow pump.

Note that the amount of chloride is, despite the addition of 7 mg of chlorides /l in the example, a positive amount for health, because it is always lower, in any added spring water, than the maximum limits established by the WHO and present in Legislative Decree 31/2001, which establishes chlorides at 250 mg /l for drinking water and 200 mg /l for natural mineral waters.

In short, lithium has an anti-aging effect, maintaining the functionality or enhancing the functional capacity of the target organs. There are mineral waters on the market that are naturally enriched with lithium, thanks to their path in the subsoil, but generally the final concentration of lithium is much lower than that capable of exhibiting detectable biological effects. This invention relates to a new product comprising water artificially enriched with lithium such as to achieve an effective dose following consumption and an example of a process aimed at its preparation.

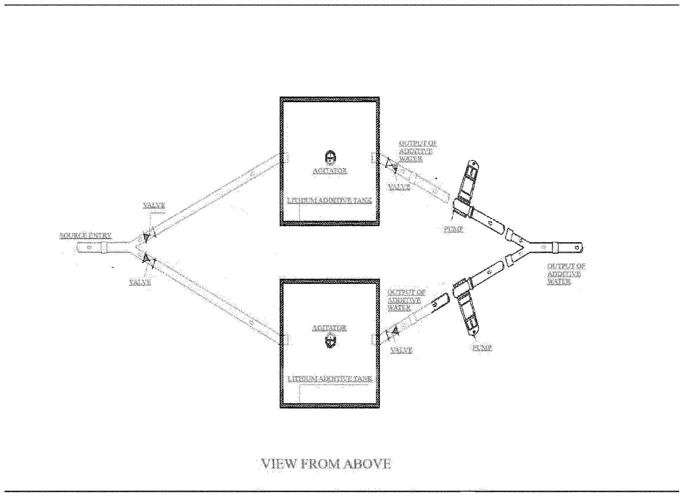

## Claims

1. A new product, comprising water artificially enriched with lithium and intended for human consumption

2. A product according to claim 1 where the lithium concentration is between 0.1 mg/litre and 10 mg/litre, preferably 1 mg/litre.

3. A process for the preparation of the product according to claims 1-2.

4. A process according to claim 3 where the lithium enrichment occurs by means of the parallel chamber system.

5. Use of the product according to claims 1-2 and obtained according to claims 3 - 4

VIEW FROM ABOVE

Europäisches
Patentamt
European
Patent Office
Office européen
des brevets

## EUROPEAN SEARCH REPORT

**Application Number**

EP 24 02 0241

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DOBOSY PÉTER ET AL: "Lithium concentration in tap water, bottled mineral water, and Danube River water in Hungary", SCIENTIFIC REPORTS, [Online] vol. 13, no. 1, 2 August 2023 (2023-08-02), XP093234304, US ISSN: 2045-2322, DOI: 10.1038/s41598-023-38864-6 Retrieved from the Internet: URL:https://www.nature.com/articles/s41598-023-38864-6> [retrieved on 2024-12-13] | 1,2,5 | INV. A61K33/00 A61K33/14 C01D15/00 C22B26/00 B01D61/00 B01F25/00 C01D3/00 |
| Y | * pg 2, 2nd and 3rd paragraph, pg4, 2nd paragraph * | 1,2,5 | |
| X | BROBERG KARIN ET AL: "Lithium in Drinking Water and Thyroid Function", ENVIRONMENTAL HEALTH PERSPECTIVES., vol. 119, no. 6, 1 June 2011 (2011-06-01), pages 827-830, XP093234331, US ISSN: 0091-6765, DOI: 10.1289/ehp.1002678 | 1,2,5 | |
| Y | * pg 1, left column * | 1,2,5 | TECHNICAL FIELDS SEARCHED (IPC) A61K C01G C22B C01D B01F B01D |
| X | WO 2012/007387 A1 (MEDESIS PHARMA [FR]; MAUREL JEAN-CLAUDE [FR]) 19 January 2012 (2012-01-19) | 1,5 | |
| Y | * claims 1, 6, 7 * | 1,2,5 | |

The present search report has been drawn up for all claims

| Place of search | Date of completion of the search | Examiner |
|---|---|---|
| The Hague | 17 December 2024 | Duez-Weigelt, S |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&  : member of the same patent family, corresponding
    document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1, 2(completely); 5(partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

**Application Number**

**EP 24 02 0241**

The Search Division considers that the present European patent application does not comply with the requirements of unity of invention and relates to several inventions or groups of inventions, namely:

1. claims: 1, 2(completely); 5(partially)

   product comprising water artificially enriched with lithium and intended for human consumption
   ---

2. claims: 3, 4(completely); 5(partially)

   a process for the preparation of the product comprising water artificially enriched with lithium and intended for human consumption
   ---

## ANNEX TO THE EUROPEAN SEARCH REPORT
## ON EUROPEAN PATENT APPLICATION NO.

EP 24 02 0241

This annex lists the patent family members relating to the patent documents cited in  the above-mentioned European search report.
The members are as contained in the European Patent Office EDP file on
The European Patent Office is in no way liable for these particulars which are merely given for the purpose of information.

17-12-2024

| Patent document cited in search report | Publication date | Patent family member(s) | Publication date |
|---|---|---|---|
| WO 2012007387    A1 | 19-01-2012 | NONE | |

For more details about this annex : see Official Journal of the European Patent Office, No. 12/82